(19) Europäisches Patentamt
European Patent Office
Office européen des brevets

(11) **EP 2 108 391 B1**

(12) **EUROPÄISCHE PATENTSCHRIFT**

(45) Veröffentlichungstag und Bekanntmachung des Hinweises auf die Patenterteilung:
**27.04.2011 Patentblatt 2011/17**

(51) Int Cl.:
*A61M 1/34* *(2006.01)*   *A61M 1/36* *(2006.01)*

(21) Anmeldenummer: **09009766.8**

(22) Anmeldetag: **28.04.2005**

(54) **Verfahren und Vorrichtung zur Überwachung der Zufuhr von Substitutionsflüssigkeit während einer extrakorporalen Blutbehandlung**

Method and device for supervising the supply of substitution fluid during an extracorporeal blood treatment

Méthode et dispositif pour surveiller l'adduction de liquide de substitution pendant un traitement extracorporel du sang

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **11.05.2004 DE 102004023080**

(43) Veröffentlichungstag der Anmeldung:
**14.10.2009 Patentblatt 2009/42**

(60) Teilanmeldung:
**10007776.7 / 2 238 996**

(62) Dokumentnummer(n) der früheren Anmeldung(en) nach Art. 76 EPÜ:
**05009278.2 / 1 595 560**

(73) Patentinhaber: **Fresenius Medical Care Deutschland GmbH**
**61352 Bad Homburg v.d.H. (DE)**

(72) Erfinder: **Kopperschmidt, Pascal, Dr.**
**97456 Dittelbrunn (DE)**

(74) Vertreter: **Luderschmidt, Schüler & Partner Patentanwälte**
**John-F.-Kennedy-Strasse 4**
**65189 Wiesbaden (DE)**

(56) Entgegenhaltungen:
**EP-A- 1 348 458   WO-A-00/51664**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung bezieht sich auf ein Verfahren zur Überwachung der Zufuhr von Substitutionsflüssigkeit für eine Vorrichtung zur extrakorporalen Blutbehandlung mit einem extrakorporalen Blutkreislauf, der eine erste Kammer eines durch eine Membran in die erste Kammer und eine zweite Kammer unterteilten Dialysators oder Filters einschließt, und einem Flüssigkeitssystem, das die zweite Kammer des Dialysators oder Filters einschließt. Darüber hinaus betrifft die Erfindung eine derartige Vorrichtung zur extrakorporalen Blutbehandlung mit einer Einrichtung zur Detektion der Zufuhr von Substitutionsflüssigkeit stromauf oder stromab des Dialysators oder Filters.

Zur Entfernung von harnpflichtigen Substanzen und zum Flüssigkeitsentzug werden beim chronischen Nierenversagen verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von eine semipermeablen Membran getrennt sind. Während der Behandlung strömt das Blut des Patienten durch die Blutkammer. Um das Blut effektiv von harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleinen molekularen Substanzen durch die Membran im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodiafiltration (HDF).

Bei der Hämo(dia)filtration wird ein Teil des durch die Membran abgezogenen Serums durch eine sterile Substitutionsflüssigkeit, ersetzt, die entweder stromauf des Dialysators oder stromab des Dialysators dem extrakorporalen Blutkreislauf zugeführt wird. Die Zufuhr, der Substitutionsflüssigkeit stromauf des Dialysators wird auch als Predilution und die Zufuhr stromab des Dialysators als Postdilution bezeichnet.

Es sind Vorrichtungen zur Hämo(dia)filtration bekannt, bei denen die Dialysierflüssigkeit online aus Frischwasser und Konzentrationen und die Substitutionsflüssigkeit online aus der Dialysierflüssigkeit hergestellt werden.

Bei den bekannten Hämo(dia)filtrationsvorrichtungen wird die Substitutionsflüssigkeit dem extrakorporalen Blutkreislauf von dem Flüssigkeitssystem der Maschine über eine Substitutionsflüssigkeitsleitung zugeführt. Bei der Predilution führt die Substitutionsflüssigkeitsleitung zu einer Anschlussstelle an der arteriellen Blutleitung stromauf des Dialysators oder Filters, während bei der Postdilution die Substitutionsflüssigkeitsleitung zu einer Anschlussstelle an der venösen Blutleitung stromab des Dialysators führt. Die Substitutionsflüssigkeitsleitung weist im Allgemeinen einen Konnektor auf, mit dem sie entweder an die venöse oder arterielle Blutleitung angeschlossen werden kann. Zum Unterbrechen der Flüssigkeitszufuhr ist an der . Substitutionsflüssigkeitsleitung eine Klemme oder dgl. vorgesehen. Ein derartiges Hämo(dia)filtrationsgerät ist beispielsweise aus der EP-A-0 189 561 bekannt.

Die Überwachung der Blutbehandlung setzt die Kenntnis voraus, ob die Substitutionsflüssigkeit stromauf oder stromab des Dialysators oder Filters dem extrakorporalen Blutkreislauf zugeführt wird. Beispielsweise spielt die Pre- und Postdilution eine Rolle für die auf einer Leitfähigkeitsmessung beruhenden Online-Clearance-Messung (OCM), da die Leitfähigkeit der Dialysierflüssigkeit stromab des Dialysators davon abhängig ist, ob eine Pre- oder Postdilution erfolgt.

Die EP-A-1 348 458 A1 beschreibt ein Verfahren und eine Vorrichtung zur Überwachung der Zufuhr von Substitutionsflüssigkeit für eine extrakorporale Blutbehandlungsvorrichtung. Für die Detektion der Zufuhr von Substitutionsflüssigkeit stromauf oder stromab des Dialysators oder Filters wird die Laufzeit der Druckwellen einer in der Substitutionsflüssigkeitsleitung angeordneten Substituatpumpe gemessen. Die Zufuhr von Substitutionsflüssigkeit stromauf oder stromab des Dialysators oder Filters wird auf der Grundlage der Laufzeitmessung erkannt. Das bekannte Verfahren setzt den Einsatz einer Druckwellen erzeugenden Substituatpumpe voraus.

Die DE 101 15 991 C1 beschreibt eine Vorrichtung zum Erkennen von Stenosen in einem Schlauchleitungssystem. Die Druckschrift schlägt vor, auf eine Stenose (Engstelle) im Schlauchleitungssystem bei einer Änderung des Frequenzspektrums einer sich in dem Schlauchleitungssystem ausbreitenden oszillierenden Drucksignals zu schließen. Das Funktionsprinzip der bekannten Vorrichtung beruht darauf, dass die Ursache für die Änderung des dynamischen Verhaltens des Schlauchleitungssystems die Compliance des Leitungssystems ist, d. h. das elastische Nachgeben unter Druck.

Der Erfindung liegt die Aufgabe zu Grunde, ein Verfahren anzugeben, das die Erkennung von Pre- und Postdilution mit hoher Zuverlässigkeit erlaubt. Darüber hinaus ist eine Aufgabe der Erfindung, eine Vorrichtung zur extrakorporalen Blutbehandlung anzugeben, die über eine Einrichtung verfügt, mit der Pre- und Postdilution zuverlässig erkannt werden kann.

Die Lösung dieser Aufgaben erfolgt erfindungsgemäß mit den in den Patentansprüchen 1 bzw. 7 angegebenen Merkmalen.

Die Erfindung beruht auf einer Messung des Drucks im Flüssigkeitssystem stromab des Dialysators oder Filters.

Die Erfindung setzt den Einsatz einer Druckwellen erzeugenden Substituatpumpe voraus. Die Zufuhr von Substitutionsflüssigkeit stromauf oder stromab des Dialysators oder Filters wird auf der Grundlage des Vergleichs des sich im Blutkreislauf fortpflanzenden oszillierenden Drucksignals, das auf die Substituatpumpe zurückzuführen ist, mit einem charakteristischen Referenzsignal erkannt. Das erfindungsgemäße Verfahren und die Vorrichtung beruhen darauf, dass das Frequenzspektrum des oszillierenden Drucksignals davon abhängig ist, ob sich das Drucksignal bei Predilution über den Dialysator oder bei Postdilution nicht über den Dialysator fortpflanzt. Auch ändert sich die Amplitude der Druckpulse in Abhängigkeit von Pre- oder Postdilution.

Bei den bekannten Blutbehandlungsvorrichtungen wird das sich im extrakorporalen Blutkreislauf fortpflanzende oszillierende Drucksignal der Substituatpumpe von weiteren Drucksignalen überlagert, die beispielsweise auf die im Blutkreislauf stromauf des Dialysators oder Filters angeordnete Blutpumpe oder im Flüssigkeitssystem angeordnete Einrichtungen zurückzuführen sind, zu denen beispielsweise die Konzentratpumpe, Ultrafiltrationspumpe oder Bilanzkammern zählen. Daher wird aus dem im Blutkreislauf gemessenen Drucksignal das auf die Substituatpumpe zurückzuführende Drucksignal gewonnen. Zur Vermeidung von Messfehlern kann der Dialysator oder Filter auch vom Flüssigkeitssystem abgetrennt werden.

Eine bevorzugte Ausführungsform, die eine Detektion der Zufuhr von Substitutionsflüssigkeit mit besonders hoher Zuverlässigkeit erlaubt, sieht als charakteristisches Referenzsignal das Signal der im extrakorporalen Blutkreislauf stromauf des Dialysators oder Filters angeordneten Blutpumpe vor, die ebenfalls oszillierende Drucksignale erzeugt. Diese Ausführungsform geht von der Annahme aus, dass sich bei Predilution Oszillation der Drucksignale von Blutpumpe und Substituatpumpe, die sich über den Dialysator und das Schlauchsystem fortpflanzen, nur unwesentlich von einander unterscheiden. Bei Postdilution hingegen unterscheiden sich Oszillation der Drucksignale von Substituatpumpe und Blutpumpe deutlich, da sich die Drucksignale der Substituatpumpe nicht über den Dialysator fortpflanzen. Damit ist nicht nur ein relatives, sondern auch absolutes Unterscheidungsmerkmal zwischen Pre- und Postdilution gegeben.

Zur Gewinnung der Drucksignale der Blutpumpe und Substituatpumpe wird vorzugsweise eine Fourier-Analyse des stromab des Dialysators oder Filters gemessenen Drucksignals durchgeführt. Aus den Drucksignalen der Substituatpumpe und Blutpumpe werden vorzugsweise die Amplituden von mindestens zwei Oberschwingungen ermittelt. Aus mindestens einem Quotienten aus mindestens einer Oberschwingung höherer Ordnung und mindestens einer Oberschwingung niedrigerer Ordnung der Substituatpumpe wird mindestens ein erster Bewertungsfaktor und aus mindestens einem Quotienten aus mindestens einer Oberschwingung höherer Ordnung und mindestens einer Oberschwingung niedrigerer Ordnung der Blutpumpe wird mindestens ein zweiter Bewertungsfaktor ermittelt.

In der Praxis hat sich als ausreichend erwiesen, wenn mit der Fourier-Transformation die Koeffizienten der zweiten oder höheren Harmonischen des Drucksignals der Blutpumpe bzw. Substituatpumpe auf den Koeffizienten der ersten Harmonischen des Drucksignals der Blutpumpe bzw. Substituatpumpe normiert werden, um die beiden Bewertungsfaktoren zu bilden.

Auf eine Postdilution wird dann geschlossen, wenn die Differenz zwischen dem ersten. Bewertungsfaktor und dem zweiten Bewertungsfaktor größer als ein vorgegebener Grenzwert ist, während auf eine Predilution geschlossen wird, wenn die Differenz zwischen dem ersten Bewertungsfaktor und dem zweiten Bewertungsfaktor kleiner als der vorgegebene Grenzwert ist.

Die Einrichtung zur Detektion der Zufuhr von Substitutionsflüssigkeit der erfindungsgemäßen extrakorporalen Blutbehandlungsvorrichtung weist eine Messeinheit zum Messen des Drucks im Blutkreislauf stromab des Dialysators oder Filters und eine Auswerteinheit auf, die wiederum Mittel zur Gewinnung des auf die Substituatpumpe zurückzuführenden oszillierenden Drucksignals, Mittel zum Vergleich des oszillierenden Drucksignals mit einem charakteristischen Referenzsignal und Mittel zur Detektion von Pre- oder Postdilution auf der Grundlage des Vergleichs des oszillierenden Drucksignals der Substituatpumpe mit dem charakteristischen Referenzsignal aufweist.

Eine Unterscheidung zwischen Pre- oder Postdilution kann zu Beginn der Dialysebehandlung allein mit der Auswertung des Drucksignals der Substituatpumpe, vorzugsweise aber in Verbindung mit dem Drucksignal der Blutpumpe getroffen werden.

Außer der Messeinheit zum Messen des Drucks im extrakorporalen Kreislauf stromab des Dialysators oder Filters, die im allgemeinen Bestandteil der bekannten Blutbehandlungsvorrichtungen ist, werden weitere Hardware-Komponenten nicht benötigt.

Das erfindungsgemäße Verfahren und die erfindungsgemäße Vorrichtung finden in vorteilhafter Weise bei der Bestimmung der Dialysedosis mittels des sog. Online-Clearance-Monitoring (OCM) und bei einer Substituatvorgabe in Abhängigkeit von dem Blutfluss Verwendung. Das Verfahren und die Vorrichtung liefern darüber hinaus eine Entscheidungshilfe in allen relevanten die Dialyse betreffenden Parametern, wenn eine Unterscheidung zwischen postdilutiver und predilutiver Substituatgabe notwendig ist. Das Verfahren und die Vorrichtung können auch Anwendung finden bei der Bestimmung des Blutflusses, der Bestimmung der Shunt- oder Fistelrezirkulation, in der Überwachung des relativen Blutvolumens bzw. Hämatokrits, der Ermittlung der Füllvolumina des Dialysators oder Filters sowie der Erkennung des venösen Nadeltyps.

Die meisten für die Detektionseinrichtung erforderlichen Komponenten sind im Allgemeinen in den bekannten Blutbehandlungsvorrichtungen bereits vorhanden. Beispielsweise kann auf den venösen Drucksensor zum Messen des Drucks im Blutkreislauf stromab des Dialysators oder Filters zurückgegriffen werden. Auch steht eine Mikroprozessorsteuerung zur Verfügung. Damit ist der apparative Aufwand relativ gering.

Im Folgenden werden die beiden alternativen Ausführungsforrnen des erfindungsgemäßen Verfahrens sowie der erfindungsgemäßen Blutbehandlungsvorrichtung unter Bezugnahme auf die Figuren näher erläutert.

Es zeigen:

Figur 1    eine erste Ausführungsform einer erfindüngsgemäßen Vorrichtung zur extrakorporalen Blutbehandlung mit einer Einrichtung zur Detektion von Pre- und Postdilution in stark vereinfachter schematischer Darstellung,

Figur 2 A    eine prinzipielle Darstellung des zeitlichen Verlaufs des Drucks im extrakorporalen Blutkreislauf stromab des Dialysators oder Filters bei Postdilution,

Figur 2 B    eine prinzipielle Darstellung des zeitlichen Verlaufs des venösen Drucks bei Predilution,

Figur 3 A    den während einer in vitro HDF-Dialysebehandlung gemessenen mittleren venösen Druck als Funktion der Zeit bei Postdilution,

Figur 3 B    den mittleren venösen Druck bei Predilution,

Figur 4    eine zweite Ausführungsform der erfindungsgemäßen extrakorporalen Blutbehandlungsvorrichtung in stark vereinfachter schematischer Darstellung,

Figur 5 A    das Frequenzspektrum der auf die Substituatpumpe und die Blutpumpe zurückzuführenden Druckwellen im extrakorporalen Blutkreislauf und eine Tabelle mit normierten Koeffizienten der Drucksignale der Blutpumpe und der Substituatpumpe bei Postdilution,

Figur 5 B    das Frequenzspektrum der auf die Substituatpumpe und die Blutpumpe zurückzuführenden Druckwellen im extrakorporalen Blutkreislauf und eine Tabelle mit normierten Koeffizienten der Drucksignale der Blutpumpe und der Substituatpumpe bei Predilution.

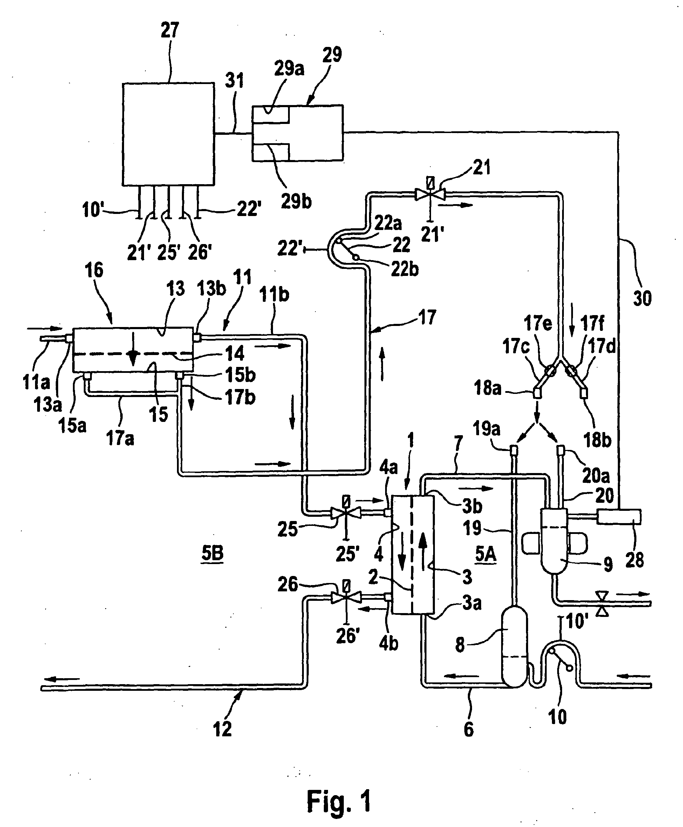

Figur 1 zeigt eine vereinfachte schematische Darstellung der wesentlichen Komponenten einer Hämo(dia)filtrationsvorrichtung zusammen mit einer Einrichtung zur Detektion der Zufuhr von Substitutionsflüssigkeit in den extrakorporalen Blutkreislauf stromauf oder stromab des Dialysators oder Filters. Wenn nachfolgend von einem Dialysator die Rede ist, wird darunter auch ein Filter verstanden.

Die Hämo(dia)filtrationsvorrichtung weist einen Dialysator 1 auf, der durch eine semipermeable Membran 2 in eine von Blut durchflossene erste Kammer 3 und eine von Dialysierflüssigkeit durchflossene zweite Kammer 4 getrennt ist. Die erste Kammer 3 ist in einen extrakorporalen Blutkreislauf 5A geschaltet, während die zweite Kammer 4 in das Flüssigkeitssystem 5B der Hämo(dia)filtrationsvorrichtung geschaltet ist.

Der extrakorporale Blutkreislauf 5A umfasst eine arterielle Blutleitung 6, die zu dem Einlass 3a der Blutkammer 3 führt, und eine venöse Blutleitung 7, die von dem Auslass 3b der Blutkammer 3 des Dialysators 1 abgeht. Zur Eliminierung von Luftblasen sind in die arterielle Blutleitung 6 eine arterielle Tropfkammer 8 und in die venöse Blutleitung 7 eine venöse Tropfkammer 9 geschaltet. Das Blut des Patienten wird durch die Blutkammer des Dialysators mittels einer arteriellen Blutpumpe 10, insbesondere Rollenpumpe gefördert, die an der arteriellen Blutleitung 6 angeordnet ist.

Das Flüssigkeitssystem 5B umfasst eine Dialysierflüssigkeitszuführleitung 11, die zu dem Einlass 4a der Dialysierflüssigkeitskammer 4 führt, und eine Dialysierflüssigkeitsabführleitung 12, die von dem Auslass 4b der Dialysierflüssigkeitskammer 4 des Dialysators 1 abgeht. Über die Dialysierflüssigkeitszuführleitung 11 strömt frische Dialysierflüssigkeit aus einer nicht dargestellten Dialysierflüssigkeitsquelle in die Dialysierflüssigkeitskammer, während die verbrauchte Dialysierflüssigkeit aus der Dialysierflüssigkeitskammer über die Dialysierflüssigkeitsabführleitung 12 zu einem nicht dargestellten Abfluss abgeführt wird. Die in den Hämo(dia)filtrationsvorrichtungen im Allgemeinen vorgesehene Bilanzierungseinrichtung zur Bilanzierung frischer gegen verbrauchte Dialysierflüssigkeit ist der besseren Übersichtlichkeit halber nicht dargestellt. Auch sind zusätzliche Einrichtungen zum Reinigen und Spülen des Systems nicht dargestellt.

Die Dialysierflüssigkeitszuführleitung 11 umfasst einen ersten Abschnitt 11 a, der zu dem Einlass 13a einer ersten Kammer 13 eines durch eine Membran 14 in die erste Kammer und eine zweite Kammer 15 unterteilten Sterilfilters 16 führt, und einen zweiten Abschnitt 11b, der von dem Auslass 13b der ersten Kammer 13 des Filters 16 abgeht und zu dem Einlass 4a der Dialysierflüssigkeitskammer 4 führt.

Während der Dialysebehandlung kann z. B. Dialysierflüssigkeit aus dem Flüssigkeitssystem 5B als Substitutionsflüssigkeit über eine Substitutionsflüssigkeitsleitung 17 dem extrakorporalen Blutkreislauf 5A zugeführt werden. Die Flüssigkeitsleitung 17 weist an beiden Enden jeweils zwei Leitungsabschnitte 17a, 17b, 17c, 17d auf. Der Leitungsabschnitt 17a ist mit dem ersten Auslass 15a des Sterilfilters 16 verbunden, während an den Leitungsabschnitten 17c und 17d jeweils ein Konnektor 18a, 18b angeschlossen ist. Mit den beiden Konnektoren 18a, 18b kann die Substitutionsflüssigkeitsleitung 17 an eine zu der arteriellen Tropfkammer 8 führende Anschlussleitung 19 bzw. eine zu der venösen Tropfkammer 9 führende Anschlussleitung 20 angeschlossen werden. Die Anschlussleitungen 19, 20 verfügen dafür über entsprechende Anschlussstücke 19a, 20a. Auf den Schlauchleitungsabschnitten 17c, 17d sitzen Schlauchklemmen 17e, 17f, mit denen wahlweise eine Flüssigkeitsverbindung nur zu der arteriellen oder venösen Tropfkammer 8, 9 hergestellt werden kann. Es ist aber auch möglich, das die Substitutionsflüssigkeitsleitung 17 mit beiden Anschlussleitungen 19, 20 verbunden und beide Schlauchklemmen 17e, 17f offen sind.

Zum Abklemmen der Substitutionsflüssigkeitsleitung 17 ist stromab des Sterilfilters 16 ein Absperrorgan 21, beispielsweise eine Schlauchklemme vorgesehen, die vorzugsweise elektromagnetisch betätigt wird. Die Substitutionsflüssigkeit wird mittels einer Okklusionspumpe, insbesondere Rollenpumpe 22 gefördert, in die die Substitutionsflussigkeitsleitung 17 eingelegt ist. Derartige Rollenpumpen gehören zum Stand der Technik. Sie verfügen über mehrere Rollen 22a, 22b, mit denen der Querschnitt der Schlauchleitung zur Förderung der Flüssigkeit verringert werden kann. Dadurch entstehen Druckwellen, die sich in beiden Richtungen über die Substitutionsflüssigkeitsleitung fortpflanzen können. Es sei bemerkt, dass eine Druckwellen erzeugende Okklusionspumpe als Substituatpumpe bei der ersten Ausführungsform der erfindungsgemäßen Blutbehandlungsvorrichtung nicht erforderlich ist. Vielmehr kann zur Förderung der Substitutionsflüssigkeit auch eine Substituatpumpe Verwendung finden, die Druckwellen nicht erzeugt. Darin liegt ein Vorteil dieser Ausführungsform.

Zur Abkopplung des Dialysators 1 von dem Flüssigkeitssystem 5B sind in der Dialysierflüssigkeitszuführleitung 11 stromauf und in der Diatysierflussigkeitsabführleitung 12 stromab des Dialysators 1 jeweils ein elektromagnetisch betätigbares Absperrorgan 25, 26 vorgesehen.

Die Blutpumpe 10, die Substituatpumpe 22 sowie die Absperrorgane 21, 25 und 26 sind über Steuerleitungen 10', 22', 21', 25' und 26' mit einer zentralen Steuer- und Regeleinheit 27 verbunden, von der die einzelnen Komponenten unter Berücksichtigung der vorgegebenen Behandlungsparameter angesteuert werden.

Zum Betrieb der Hämo(dia)filtrationsvorrichtung als Hämodialysevorrichtung wird das Absperrorgan 21 geschlossen, wobei Dialysierflüssigkeit durch die Dialysierflüssigkeitskammer 4 des Dialysators strömt. Zum Betrieb der Hämo(dia)filtrationsvorrichtung als Hämodiafiltrationsvorrichtung wird das Absperrorgan 21 geöffnet, so dass aus dem Sterilfilter 16 sterile Dialysierflüssigkeit als Substitutionsflüssigkeit wahlweise in die arterielle Tropfkammer 8 (Predilution) oder venöse Tropfkammer 9 (Postdilution) strömt. Es ist aber auch ein Betrieb der Hämo(dia)filtrationsvorrichtung nur als Hämofiltrationsvorrichtung möglich, wenn der Zufluss von Dialysierflüssigkeit in die Dialysierflüssigkeitskammer 4 des Dialysators unterbrochen wird. Zur Unterbrechung der Flüssigkeitszufuhr wird das Absperrorgan 25 stromauf des Dialysators geschlossen.

Die Einrichtung zur Detektion von Pre- und Postdilution weist eine Steuereinheit auf, die Teil der zentralen Steuer- und Regeleinheit 27 der Blutbehandlungsvorrichtung ist. Darüber hinaus weist die Detektionseinrichtung eine Messeinheit 28 zum Messen des Drucks im extrakorporalen Blutkreislauf stromab des Dialysators 3 und eine Auswerteinheit 29 auf. Als Messeinheit ist ein an der Tropfkammer 9 angebrachter Drucksensor 28 vorgesehen, der ein von dem venösen Druck abhängiges elektrisches Drucksignal erzeugt. Das Drucksignal des Drucksensors 28 wird über eine Datenleitung 30 der Auswerteinheit 29 zugeführt, die wiederum über eine Datenleitung 31 mit der Steuereinheit 27 in Verbindung steht. Die Auswerteinheit 29 weist einen Tiefpassfilter 29a zum Filtern des Drucksignals des Drucksensors 28 und einen Komparator 29b auf, mit dem das Drucksignal mit einem vorgegebenen oberen und unteren Grenzwert verglichen wird.

Nachfolgend wird die Detektion der Zufuhr von Substitutionsflüssigkeit stromauf oder stromab des Dialysators im einzelnen beschrieben. Die Steuereinheit 27 der Detektionseinrichtung schaltet vorzugsweise zu Beginn der Blutbehandlung für die Einleitung der Messung die bereits laufende Substituatpumpe 22 ab. Nach Ablauf eines vorgegebenen Zeitintervalls schaltet die Steuereinheit 27 die Substituatpumpe 22 wieder an. Während die Substituatpumpe ausgeschaltet ist, misst der Drucksensor 28 den venösen Druck. Das mit dem Tiefpassfilter 29a gefilterte venöse Drucksignal des Sensors 28 wird in dem Komparator 29b der Auswerteinheit 29 mit einem oberen und unteren Grenzwert verglichen, um entweder nach dem Abschalten der Substituatpumpe einen Druckanstieg und nach dem Einschalten der Substituatpumpe einen Druckabfall oder nach dem Abschalten der Substituatpumpe einen Druckabfall und nach dem Einschalten der Substituatpumpe einen Druckanstieg zu detektieren:

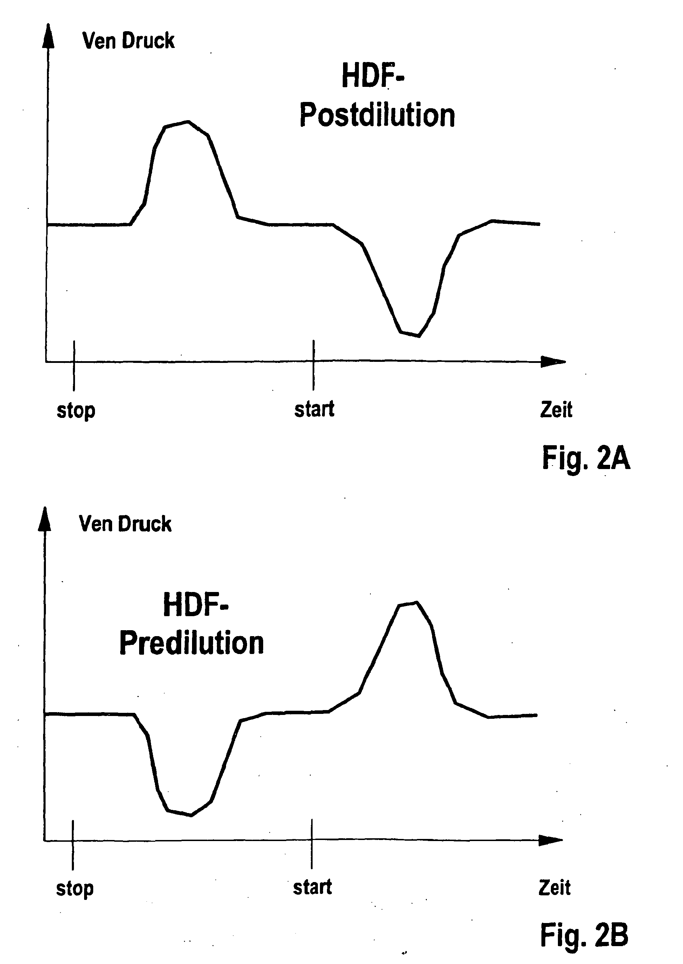

Figur 2A zeigt den prinzipiellen zeitlichen Verlauf des venösen Drucks bei Postdilution und Figur 2B den zeitlichen Verlauf des Drucks bei Predilution. Deutlich ist zu erkennen, dass bei Postdilution der Druck zunächst ansteigt, um anschließend abzufallen. Bei Predilution fällt der Druck zunächst ab, um dann anzusteigen. Auf der Grundlage dieser beiden charakteristischen Verläufe erkennt die Auswerteinheit, ob Postdilution oder Predilution vorliegt. Das Ergebnis kann auf einer nicht dargestellten Anzeigeeinheit optisch und/oder akustisch angezeigt werden.

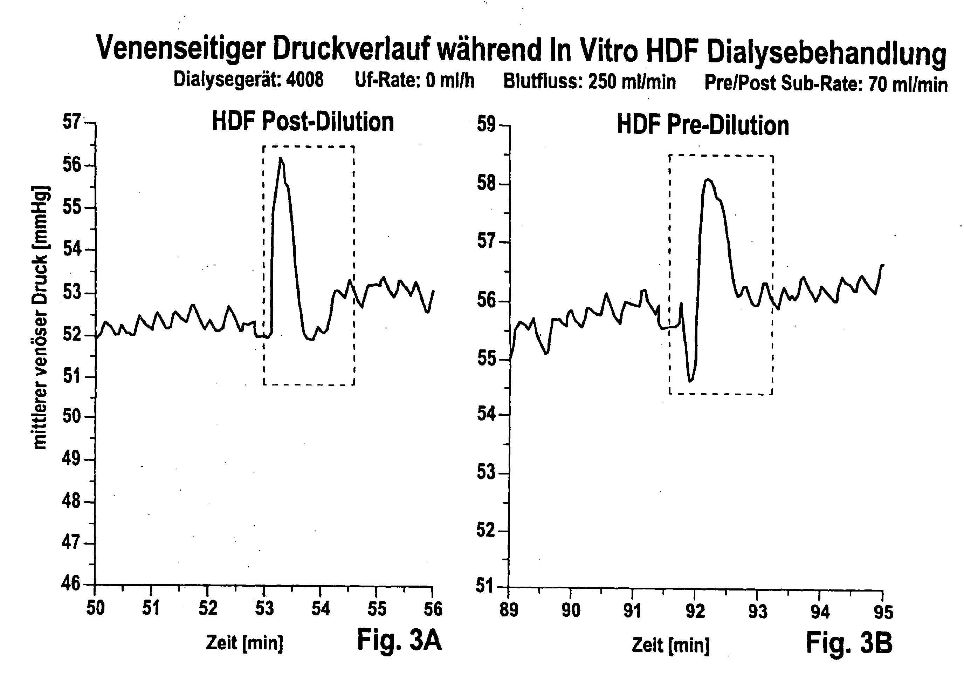

Die Figuren 3A und 3B zeigen den venösen Druck als Funktion der Zeit für den Fall, dass das vorgegebene Zeitintervall, in dem die Substituatpumpe ausgeschaltet ist, relativ lang ist. Wenn ein kürzeres Zeitintervall vorgegebenen wird, liegen die Maxima bzw. Minima näher bei einander. Zur Einleitung der Messung wird die bereits eingeschaltete Substituatpumpe für ein vorgegebenes Zeitintervall ausgeschaltet. Grundsätzlich ist es aber auch möglich, die zunächst ausgeschaltete Substituatpumpe für ein vorgegebenes Zeitintervall einzuschalten.

Der in den Figuren 2A und 2B gezeigte charakteristische Verlauf des Drucksignals ist auf die veränderte Viskosität des Blutes bei Pre- oder Postdilution zurückzuführen. Bei der Postdilution wird die Viskosität des Blutes aufgrund der Zufuhr von Substitutionsflüssigkeit in der venösen Blutleitung 7 stromab der Tropfkammern 9 verringert. Wird die Substituatpumpe 22 angehalten, erhöht sich die Viskosität des Bluts in diesem Abschnitt der venösen Blutleitung. Folglich tritt an der venösen Nadel ein erhöhter Druckabfall auf. Dadurch steigt der venöse Druck an. Wenn die Substituatpumpe wieder eingeschaltet wird, verringert sich die Viskosität des Blutes in der venösen Blutleitung 7 stromab der Tropfkammer 9, so dass der venöse Druck abfällt. Folglich erfolgt nach dem Stop der Substituatpumpe ein Druckpuls mit positivem Vorzeichen und nach dem Start der Pumpe ein Druckpuls mit negativem Vorzeichen. (Fig. 2A). Die Pulsbreite hängt vom Verhältnis des Blutvolumens zwischen der Dialysierflüssigkeitskammer 3 des Dialysators 1 und der Tropfkammer 9 sowie dem Blutfluss ab. Bei Predilution hingegen kehrt sich nach dem Anhalten und Einschalten der Substituatpumpe die Reihenfolge der Vorzeichen des Druckpulses um. Es erfolgt beim Stop der Pumpe ein Druckabfall und nach dem Start der Pumpe ein Druckanstieg (Figur 2B).

Die Figuren 3A und 3B zeigen den mittleren venösen Druck [mmHg] als Funktion der Zeit während einer in vitro HDF-Dialysebehandlung, wobei der Blutfluss auf 250 ml/min, die Substitutionsrate für Pre- und Postdilution auf 70 ml/min und die Ultrafiltrationsrate auf 0 ml/h eingestellt ist. In der Praxis zeigt sich, dass die Amplituden des Druckanstiegs oder Druckabfalls unterschiedlich sind. Der Druckanstieg oder Druckabfall ist aber sowohl bei der Pre- als auch Postdilution deutlich zu erkennen. Die vorgegebenen oberen und unteren Grenzwerte sind derart einzustellen, dass sie unterhalb bzw. oberhalb der Maxima bzw. Minima liegen. Andererseits sollten die oberen und unteren Grenzwerte aber oberhalb bzw. unterhalb der dem Drucksignal überlagerten Druckschwankungen liegen.

Aus den positiven bzw. negativen Druckpulsen kann auch auf die Fistelrezirkulation geschlossen werden. Eine Wiederholung der Druckpulsfolge, die sich in der venösen Blutleitung 7 mit dem venösen Drucksensor 28 nachweisen lässt, in der arteriellen Blutleitung 6, die sich mit einem arteriellen Drucksensor nachweisen lässt, deutet auf eine Rezirkulation hin. Als Maß für die Rezirkulation dient das Verhältnis zwischen dem Integral des Drucksignals des arteriellen Drucksensors in einem vorgegebenen Zeitintervall, in dem der sich wiederholende negative bzw. positive Druckpuls liegt und dem Integral des Drucksignals des venösen Drucksensors 28 in einem vorgegebenen Zeitintervall, in dem der negative bzw. positive Druckpuls liegt. Die Rezirkulation Rez [%] berechnet sich nach der folgenden Gleichung:

$$Re\,z[\%] = 100 \cdot \left( \frac{\int p_{art}\,dt}{\int p_{ven}\,dt} \right)$$

Aus der Zeitdifferenz ($\Delta t$) zwischen dem Beginn und Ende der Druckpulserhöhung oder Druckpulsverringerung kann bei Kenntnis der Blutförderrate ($Q_b$) auf das Füllvolumen ($V_{PD}$) des Schlauchsegments zwischen dem Eingang 3a der Dialysierflüssigkeitskammer 3 des Dialysators 1 und der Anschlussstelle der Substitutionsflüssigkeitsleitung 17, 20 an der Tropfkammer 9 geschlossen werden. Das Volumen berechnet sich nach der folgenden Gleichung:

$$V_{PD} = Q_b \Delta t = Q_b \left( t_{Ende} - t_{Beginn} \right)$$

Darüber hinaus kann auf das Füllvolumen ($V_{PD}$) des Schlauchsegments zwischen dem Eingang 3a der Dialysierflüssigkeitskammer 3 und der venösen Nadel geschlossen werden, wenn bei der HDF-Predilution das vorgegebene Zeitintervall zwischen dem Anhalten der Substituatpumpe 22 und dem Beginn des Druckabfalls bzw. das Zeitintervall zwischen dem Einschalten der Substituatpumpe und dem Beginn des Druckanstiegs mit der Blutförderrate ($Q_b$) multipliziert wird. Bei HDF-Postdilution besteht ein analoger Zusammenhang, wobei die Zeitdifferenz zwischen dem Stop der Substituatpumpe und dem Beginn des Druckpulsanstiegs bzw. die Zeitdifferenz zwischen dem Start der Substituatpumpe und dem Beginn des Druckpulsabfalls überwacht wird.

In umgekehrter Weise kann bei Kenntnis des Füllvolumens jeweils der oben genannten Schlauchsegmente und Zeitdifferenzen der wahre Blutfluss ermittelt werden.

Ist der Zusammenhang zwischen Hämatokrit, Blutdruckabfall bzw. Blutdruckanstieg, Blutförderrate und Nadelgeometrie bekannt, so gibt die Amplitude des Druckanstiegs bzw. -abfalls Aufschluss über den Typ der Nadel. Wenn der Typ der Nadel bekannt ist, kann entsprechend auf den Hämatokrit geschlossen werden.

So kann die Änderung des Hämatokrits während der Dialysebehandlung anhand der Änderung der Amplitude der Druckpulse überwacht werden.

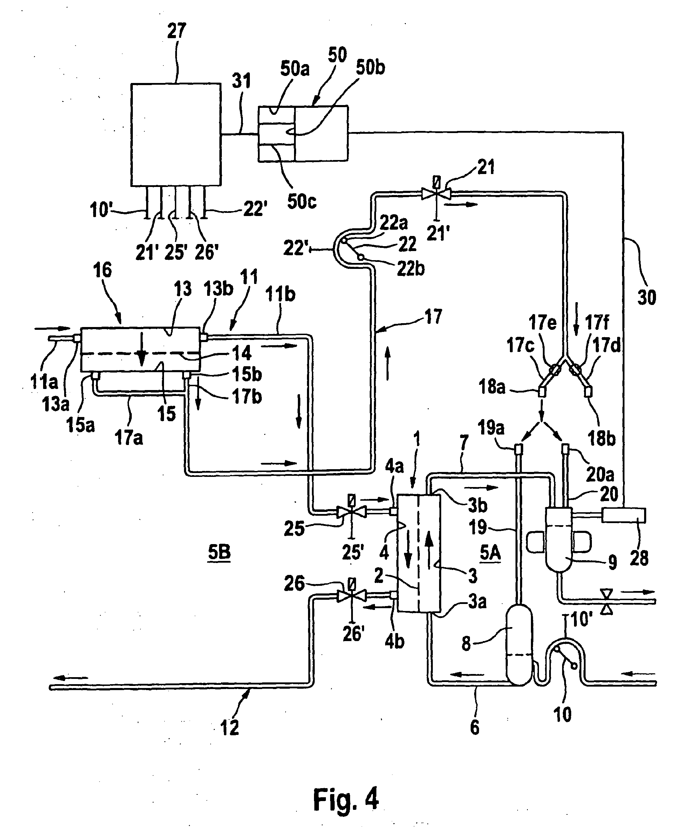

Figur 4 zeigt die alternative Ausführungsform der erfindungsgemäßen Blutbehandlungsvorrichtung in vereinfachter schematischer Darstellung. Mit Ausnahme der Einrichtung zur Detektion von Pre- oder Postdilution unterscheidet sich die Blutbehandlungsvorrichtung von Figur 4 nicht von der Blutbehandlungsvorrichtung von Figur 1. Daher werden die einander entsprechenden Komponenten auch mit den gleichen Bezugszeichen bezeichnet.

Die Detektionseinrichtung weist auch einen venösen Drucksensor 28 zum Messen des Drucks in der venösen Blutleitung 7 auf. Die Auswerteinheit 50 empfängt das venöse Drucksignal des Drucksensors 28 über die Datenleitung 30. Die Auswerteinheit 50 weist eine Einrichtung zur Fourier-Analyse 50a auf, die eine Fourier-Analyse des venösen Drucksignals durchführt.

Das Verfahren zur Detektion von Pre- oder Postdilution setzt voraus, dass die Substituatpumpe 22 und die Blutpumpe 10 Druckwellen erzeugende Pumpen sind, beispielsweise Okklusionspumpen, insbesondere Rollenpumpen. Da die Frequenz der von der Blutpumpe 10 erzeugten Druckwellen infolge der sehr viel höheren Drehzahl der Blutpumpe im Vergleich mit der Substituatpumpe sehr viel größer als die Frequenz der Druckwellen der Substituatpumpe ist, können die Druckwellen der Blutpumpe von denen der Substituatpumpe unterschieden werden. Bei der Predilution durchlaufen die Druckwellen der Substituatpumpe 22 neben dem Schlauchabschnitt der Substitutionsflüssigkeitsleitung 17, 19, 20 den Schlauchabschnitt der arteriellen Blutleitung 6 zwischen Tropfkammer 8 und Eingang 3a der Dialysierflüssigkeitskammer 3, die Dialysierflüssigkeitskammer und die venöse Blutleitung 7, bevor sie den Drucksensor 28 erreichen. Bei der Postdilution durchlaufen die Druckwellen hingegen nicht die Dialysierflüssigkeitskammer und die zugehörigen Blutleitungsabschnitte.

Die Auswirkung der Dialysierflüssigkeitskammer 3 und der dazugehörigen Blutleitungsabschnitte kann durch eine Übertragungsfunktion beschrieben werden. Wenn der Dialysator und die zugehörigen Leitungsabschnitte in dem Übertragungsweg der Druckwellen liegen, ändert sich die Dynamik der Druckpulse und deren Frequenzspektrum, insbesondere werden höhere Frequenzen stärker gedämpft.

Eine genaue Kenntnis der Übertragungsfunktion ist für die Detektion der Pre- oder Postdilution nicht erforderlich. Ausreichend ist, wenn das Drucksignal der Substituatpumpe mit dem Drucksignal der Blutpumpe ins Verhältnis gesetzt wird.

Das mit dem Drucksensor 28 gemessene Drucksignal enthält sowohl das Drucksignal der Blutpumpe 10 als auch das der Substituatpumpe 22. Die Einrichtung zur Fourier-Transformation 50a der Auswerteinheit 50 zerlegt das mit dem Drucksensor 28 gemessene Drucksignal der Blutpumpe 10 und der Substituatpumpe 22 in die auf die Blutpumpe bzw. Substituatpumpe zurückzuführenden Signalkomponenten.

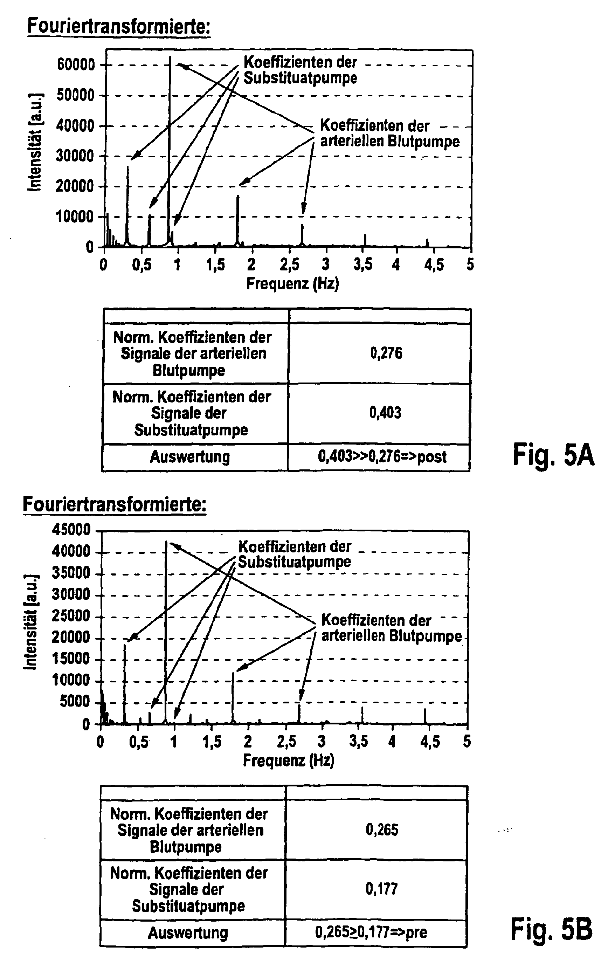

Die Figuren 5A und 5B zeigen das mit der Fourier-Transformation ermittelte Frequenzspektrum des venösen Drucksignals bei Post- bzw. Predilution. Die in den Figuren gezeigten Messwerte wurden bei einem Laborversuch ermittelt, bei dem der Blutfluss auf 300 ml/min, der Substituatfluss auf 80 ml/min und die Ultrafiltrationsrate auf 100 ml/h eingestellt wurde. In dem Frequenzspektrum sind die Koeffizienten der ersten Oberschwingung sowie der Oberschwingungen höherer Ordnung zu erkennen, die auf die Blutpumpe 10 und die Substituatpumpe 22 zurückzuführen sind.

Die Auswerteinheit 50 berechnet den Quotienten zwischen einer Harmonischen höherer Ordnung, beispielsweise der zweiten oder dritten harmonischen und dem Koeffizienten der harmonischen ersten Ordnung, d.h. den normierten Koeffizienten der spektralen Zerlegung. Der normierte Koeffizient der Substituatpumpe stellt einen ersten Bewertungsfaktor dar, während der normierte Koeffizient der Blutpumpe einen zweiten Bewertungsfaktor darstellt.
Zum Vergleichen der Bewertungsfaktoren der Substituatpumpe und der Blutpumpe weist die Auswerteinheit 50 eine weitere Einheit 50b auf. Wenn der Bewertungsfaktor der Substituatpumpe erheblich oberhalb des Bewertungsfaktors der Blutpumpe liegt, wird auf eine Postdilution geschlossen (Fig. 5A). Liegt der Bewertungsfaktor der Substituatpumpe nur oberhalb oder nahe der Substituatpumpe oder sind beide Bewertungsfaktoren sogar gleich, so wird auf eine Predilution geschlossen (Fig. 5B). Die Detektion von Pre- oder Postdilution auf der Grundlage des Vergleichs der Bewertungsfaktoren erfolgt in einer Einrichtung 50c.

Die Einrichtung 50c zum Erkennen von Pre- oder Postdilution verfügt über eine Einrichtung zur Bildung der Differenz zwischen den beiden Bewertungsfaktoren. Die Differenz wird mit einem vorgegebenen Grenzwert verglichen, der derart festgelegt ist, dass sicher zwischen Pre- oder Postdilution unterschieden werden kann. Eine Postdilution liegt vor, wenn die Differenz zwischen dem ersten und zweiten Bewertungsfaktor größer als der vorgegebene Grenzwert ist, und eine Predilution liegt vor, wenn die Differenz zwischen dem ersten und zweiten Bewertungsfaktor kleiner als der vorgegebene Grenzwert ist. Das Ergebnis kann in einer nicht dargestellten optischen und/oder akustischen Anzeigeeinheit angezeigt werden.

**Patentansprüche**

1. Verfahren zur Überwachung der Zufuhr von Substitutionsflüssigkeit für eine Vorrichtung zur extrakorporalen Blutbehandlung mit einem extrakorporalen Blutkreislauf, der eine erste Kammer eines durch eine Membran in die erste Kammer und eine zweite Kammer unterteilten Dialysators oder Filters einschließt, und einem Flüssigkeitssystem, dass die zweite Kammer des Dialysators oder Filters einschließt, wobei Substitutionsflüssigkeit stromauf oder stromab des Dialysators oder Filters mit einer Druckwellen erzeugenden Substituatpumpe zugeführt wird,
   **dadurch gekennzeichnet, dass** für die Detektion der Zufuhr von Substitutionsflüssigkeit der Druck im extrakorporalen Blutkreislauf stromab des Dialysators oder Filters gemessen wird,
   dass aus dem stromab des Dialysators oder Filters gemessenen sich im Blutkreislauf fortpflanzenden oszillierenden Drucksignals, das auf die Substituatpumpe zurückzuführende Drucksignal gewonnen wird,

wobei eine Zufuhr von Substitutionsflüssigkeit stromauf oder stromab des Dialysators oder Filters auf der Grundlage des Vergleichs des oszillierenden Drucksignals der Substituatpumpe mit einem charakteristischen Referenzsignal erkannt wird.

2.   Verfahren nach Anspruch 1, **dadurch gekennzeichnet, dass** das Blut im extrakorporalen Blutkreislauf mit einer stromauf des Dialysators oder Filters angeordneten Druckwellen erzeugenden Blutpumpe gefördert wird, und das aus dem stromab des Dialysators oder Filters gemessenen sich im Blutkreislauf fortpflanzenden oszillierenden Drucksignal das auf die Blutpumpe zurückzuführende Drucksignal gewonnen wird, wobei eine Zufuhr von Substitutionsflüssigkeit stromauf oder stromab des Dialysators oder Filters auf der Grundlage des Vergleichs der oszillierenden Drucksignale der Blutpumpe und der Substituatpumpe erkannt wird.

3.   Verfahren nach Anspruch 2, **dadurch gekennzeichnet, dass** zur Gewinnung der oszillierenden Drucksignale der Blutpumpe und der Substituatpumpe eine Fourier-Analyse des stromab des Dialysators oder Filters gemessenen Drucksignals durchgeführt wird.

4.   Verfahren nach Anspruch 3, **dadurch gekennzeichnet, dass** aus dem Drucksignal der Substituatpumpe die Amplitude von mindestens zwei Oberschwingungen der Substituatpumpe und aus dem Drucksignal der Blutpumpe die Amplitude von mindestens zwei Oberschwingungen der Blutpumpe ermittelt werden,

dass aus mindestens einem Quotienten aus mindestens einer Oberschwingung höherer Ordnung und mindestens einer Oberschwingung niedrigerer Ordnung der Substituatpumpe mindestens ein erster Bewertungsfaktor und aus mindestens einem Quotienten aus mindestens einer Oberschwingung höherer Ordnung und mindestens einer Oberschwingung niedrigerer Ordnung der Blutpumpe mindestens ein zweiter Bewertungsfaktor ermittelt werden, und

dass der mindestens eine erste und zweite Bewertungsfaktor miteinander verglichen werden.

5.   Verfahren nach Anspruch 4, **dadurch gekennzeichnet, dass** auf die Zufuhr von Substitutionsflüssigkeit stromab des Dialysators oder Filters geschlossen wird, wenn die Differenz zwischen dem ersten Bewertungsfaktor und dem zweiten Bewertungsfaktor größer als ein vorgegebener Grenzwert ist.

6.   Verfahren nach Anspruch 4, **dadurch gekennzeichnet, dass** auf die Zufuhr von Substitutionsflüssigkeit stromauf des Dialysators oder Filters geschlossen wird, wenn die Differenz zwischen dem ersten Bewertungsfaktor und dem zweiten Bewertungsfaktor kleiner als ein vorgegebener Grenzwert ist.

7.   Vorrichtung zur extrakorporalen Blutbehandlung mit einem extrakorporalen Blutkreislauf (5A), der eine erste Kammer (3) eines durch eine Membran (2) in die erste Kammer und eine zweite Kammer (4) unterteilten Dialysators (1) oder Filters einschließt, und einem Flüssigkeitssystem (5B), dass die zweite Kammer des Dialysators oder Filters einschließt, wobei eine Substitutionsflüssigkeitsleitung (17, 19, 20) zu dem extrakorporalen Blutkreislauf (5A) stromauf oder stromab des Dialysators (1) führt, in der eine Druckwellen erzeugende Substituatpumpe (22) angeordnet ist, und

einer Einrichtung zur Detektion der Zufuhr von Substitutionsflüssigkeit stromauf oder stromab des Dialysator (1) oder Filters,

**dadurch gekennzeichnet, dass**

die Einrichtung zur Detektion der Zufuhr von Substitutionsflüssigkeit aufweist:

eine Messeinheit (28) zum Messen des Drucks im extrakorporalen Blutkreislauf (5A) stromab des Dialysators (1) oder Filters, und

eine Auswerteinheit (50), die aufweist:

Mittel (50a) zur Gewinnung des auf die Substituatpumpe (22) oszillierenden Drucksignals aus dem stromab des Dialysator (1) oder Filters gemessenen sich im Blutkreislauf fortpflanzenden oszillierenden Drucksignals,

Mittel (50b) zum Vergleich des oszillierenden Drucksignals der Substituatpumpe (22) mit einem charakteristischen Referenzsignal, und

Mittel (50c) zur Erkennung der Zufuhr von Substitutionsflüssigkeit stromauf oder stromab des Dialysators (1) oder Filters auf der Grundlage des Vergleichs des oszillierenden Drucksignals der Substituatpumpe (22) mit dem charakteristischen Referenzsignal.

8.   Vorrichtung nach Anspruch 7, **dadurch gekennzeichnet, dass** in dem Blutkreislauf (5a) stromauf des Dialysators (1) oder Filters eine Druckwellen erzeugende Blutpumpe (10) zum Fördern des Bluts angeordnet ist, und das die Auswerteinheit (50) ferner aufweist:

Mittel (50a) zur Gewinnung des auf die Blutpumpe (10) zurückzuführenden oszillierenden Drucksignals aus dem stromab des Dialysators (1) oder Filters gemessenen sich im Blutkreislauf fortpflanzenden oszillierenden Drucksignals,

wobei die Mittel (50c) zur Erkennung der Zufuhr von Substitutionsflüssigkeit stromauf oder stromab des Dialysators (1) oder Filters derart ausgebildet sind, dass auf der Grundlage des Vergleichs des oszillierenden Drucksignals der Substituatpumpe (22) mit dem Drucksignal der Blutpumpe (10) die Zufuhr der Substitutionsflüssigkeit stromauf und stromab des Dialysators oder Filters erkannt wird.

**9.** Vorrichtung nach Anspruch 8, **dadurch gekennzeichnet, dass** die Mittel zur Gewinnung der oszillierenden Drucksignale der Substituatpumpe (22) und der Blutpumpe (10) eine Einrichtung zur Fourier-Analyse (50a) des stromab des Dialysators (1) oder Filters gemessenen Drucksignals aufweist.

**10.** Vorrichtung nach Anspruch 9, **dadurch gekennzeichnet, dass** die Einrichtung (50a) zur Fourier-Analyse derart ausgebildet ist,

dass aus dem oszillierenden Drucksignal der Substituatpumpe (22) die Amplitude von mindestens zwei Oberschwingungen der Substituatpumpe und aus dem oszillierenden Drucksignal der Blutpumpe (10) mindestens zwei Oberschwingungen der Blutpumpe ermittelt werden, wobei aus mindestens einem Quotienten aus mindestens einer Oberschwingung höherer Ordnung und mindestens einer Oberschwingung niedrigerer Ordnung der Substituatpumpe (22) mindestes ein erster Bewertungsfaktor und aus mindestens einem Quotienten aus mindestens einer Oberschwingung höherer Ordnung und mindestens einer Oberschwingung niedrigerer Ordnung der Blutpumpe (10) mindestens ein zweiter Bewertungsfaktor ermittelt werden, und

dass die Mittel (50b) zum Vergleich des oszillierenden Drucksignals derart ausgebildet sind, dass der mindestens eine erste und zweite Bewertungsfaktor miteinander verglichen werden.

**11.** Vorrichtung nach Anspruch 10, **dadurch gekennzeichnet, dass** die Mittel (50c) zur Erkennung der Zufuhr von Substitutionsflüssigkeit derart ausgebildet sind, dass auf die Zufuhr von Substitutionsflüssigkeit stromab des Dialysators (1) oder Filters geschlossen wird, wenn die Differenz zwischen dem ersten Bewertungsfaktor und dem zweiten Bewertungsfaktor größer als ein vorgegebener Grenzwert ist.

**12.** Vorrichtung nach Anspruch 10, **dadurch gekennzeichnet, dass** die Mittel (50c) zur Erkennung der Zufuhr von Substitutionsflüssigkeit derart ausgebildet sind, dass auf die Zufuhr von Substitutionsflüssigkeit stromauf des Dialysators (1) oder Filters geschlossen wird, wenn die Differenz zwischen dem ersten Bewertungsfaktor und dem zweiten Bewertungsfaktor kleiner als ein vorgegebener Grenzwert ist.

**Claims**

**1.** A method for supervising the supply of substitution fluid for an apparatus for extracorporeal blood treatment with an extracorporeal blood circuit, which comprises a first chamber of a dialyser or filter divided by a membrane into the first chamber and a second chamber, and a fluid system which comprises the second chamber of the dialyser or filter, wherein substitution fluid is fed upstream or downstream of the dialyser or filter with a substitute pump generating pressure waves,

**characterised in that**, for the detection of the supply of substitution fluid, the pressure in the extracorporeal blood circuit is measured downstream of the dialyser or filter,

that the pressure signal attributable to the substitute pump is obtained from the oscillating pressure signal propagated in the blood circuit and measured downstream of the dialyser or filter,

wherein a supply of substitution fluid upstream or downstream of the dialyser or filter is detected on the basis of the comparison of the oscillating pressure signal of the substitute pump with a characteristic reference signal.

**2.** The method according to claim 1, **characterised in that** the blood in the extracorporeal blood circuit is conveyed by a blood pump generating pressure waves and disposed upstream of the dialyser or filter, and that the pressure signal attributable to the blood pump is obtained from the oscillating pressure signal propagated in the blood circuit and measured downstream of the dialyser or filter, wherein a supply of substitution fluid upstream or downstream of the dialyser or filter is detected on the basis of the comparison of the oscillating pressure signals of the blood pump and the substitute pump.

**3.** The method according to claim 2, **characterised in that** a Fourier analysis of the pressure signal measured downstream of the dialyser or filter is carried out in order to obtain the oscillating pressure signals of the blood pump and the substitute pump.

**4.** The method according to claim 3, **characterised in that** the amplitude of at least two harmonic oscillations of the substitute pump is ascertained from the pressure signal of the substitute pump and the amplitude of at least two harmonic oscillations of the blood pump is ascertained from the pressure signal of the blood pump,

that at least a first evaluation factor is ascertained from at least one quotient of at least one higher-order harmonic oscillation and at least one lower-order harmonic oscillation of the substituate pump and at least a second evaluation factor is ascertained from at least one quotient of at least one higher-order harmonic oscillation and at least one lower-order harmonic oscillation of the blood pump, and

that the at least one first and second evaluation factor are compared with one another.

5. The method according to claim 4, **characterised in that** it is concluded that there is a supply of substitution fluid downstream of the dialyser or filter if the difference between the first evaluation factor and the second evaluation factor is greater than a predetermined threshold value.

6. The method according to claim 4, **characterised in that** it is concluded that there is a supply of substitution fluid upstream of the dialyser or filter if the difference between the first evaluation factor and the second evaluation factor is less than a predetermined threshold value.

7. An apparatus for extracorporeal blood treatment with an extracorporeal blood circuit (5A), which comprises a first chamber (3) of a dialyser (1) or filter divided by a membrane (2) into the first chamber and a second chamber (4), and a fluid system (5B) which comprises the second chamber of the dialyser or filter, wherein a substitution fluid line (17, 19, 20) leads to the extracorporeal blood circuit (5A) upstream or downstream of the dialyser (1), in which line a substituate pump (22) generating pressure waves is disposed, and

a device for detecting the supply of substitution fluid upstream or downstream of the dialyser (1) or filter, **characterised in that**

the device for detecting the supply of substitution fluid comprises:

a measuring unit (28) for measuring the pressure in the extracorporeal blood circuit (5A) downstream of the dialyser (1) or filter, and an evaluation unit (50), which comprises:

means (50a) for obtaining the pressure signal oscillating on the substituate pump (22) from the oscillating pressure signal propagated in the blood circuit and measured downstream of the dialyser (1) or filter, means (50b) for comparing the oscillating pressure signal of the substituate pump (22) with a characteristic reference signal, and means (50c) for detecting the supply of substitution fluid upstream or downstream of the dialyser (1) or filter on the basis of the comparison of the oscillating pressure signal of the substituate pump (22) with the characteristic reference signal.

8. The device according to claim 7, **characterised in that** a blood pump (10) for conveying the blood and generating pressure waves is disposed in the blood circuit (5a) upstream of the dialyser (1) or filter, and that the evaluation unit (50) further comprises:

means (50a) for obtaining the oscillating pressure signal attributable to the blood pump (10) from the oscillating pressure signal propagated in the blood circuit and measured downstream of the dialyser (1) or filter, wherein the means (50c) for detecting the supply of substitution fluid upstream or downstream of the dialyser (1) or filter are designed in such a way that the supply of substitution fluid upstream and downstream of the dialyser or filter is detected on the basis of the comparison of the oscillating pressure signal of the substituate pump (22) with the pressure signal of the blood pump (10).

9. The device according to claim 8, **characterised in that** the means of obtaining the oscillating pressure signals of the substituate pump (22) and the blood pump (10) comprises a device for the Fourier analysis (50a) of the pressure signal measured downstream of the dialyser (1) or filter.

10. The device according to claim 9, **characterised in that** the device (50a) for the Fourier analysis is designed in such a way

that the amplitude of at least two harmonic oscillations of the substituate pump are ascertained from the oscillating pressure signal of the substituate pump (22) and at least two harmonic oscillations of the blood pump are ascertained from the oscillating pressure signal of the blood pump (10), wherein at least a first evaluation factor is ascertained from at least one quotient of at least one higher-order harmonic oscillation and at least one lower-order harmonic oscillation of the substituate pump (22) and at least a second evaluation factor is ascertained from at least one quotient of at least one higher-order harmonic oscillation and at least one lower-order harmonic oscillation of the blood pump, and that the means (50b) for comparing the oscillating pressure signal are designed in such a way that the at least a first and second evaluation factor are compared with one another.

11. The device according to claim 10, **characterised in that** the means (50c) for detecting the supply of substitution fluid are designed in such a way that it is concluded that there is a supply of substitution fluid downstream of the dialyser (1) or filter if the difference between the first evaluation factor and the second evaluation factor is greater than a predetermined threshold value.

12. The device according to claim 10, **characterised in that** the means (50c) for detecting the supply of substitution fluid are designed in such a way that it is concluded that there is a supply of substitution fluid upstream of the dialyser (1) or filter if the difference between the first evaluation factor and the second evaluation factor is less than a predetermined threshold value.


**Revendications**

1. Procédé de surveillance de l'apport en liquide de substitution pour un appareil de traitement extracorporel du sang avec un circuit sanguin extracorporel qui comprend un premier compartiment d'un dialyseur ou d'un filtre subdivisé par une membrane en ledit premier compartiment et en un deuxième compartiment, et un système de liquide qui comprend ledit deuxième compartiment du dialyseur ou du filtre, le liquide de substitution étant alimenté en amont ou en aval du dialyseur ou du filtre par une pompe à substitut générant des ondes de pression, **caractérisé en ce que**, pour la détection de l'apport en liquide de substitution, la pression du circuit sanguin extracorporel est mesurée en aval du dialyseur ou du filtre, **en ce que** le signal de pression à retourner à la pompe à substitut est obtenu à partir du signal de pression oscillant et se propageant dans le circuit sanguin, mesuré en aval du dialyseur ou du filtre, un apport en liquide de substitution en amont ou en aval du dialyseur ou du filtre étant détecté sur la base de la comparaison du signal oscillant de pression de la pompe à substitut avec un signal de référence caractéristique.

2. Procédé selon la revendication 1, **caractérisé en ce que** le sang du circuit sanguin extracorporel est refoulé par une pompe à sang générant des ondes de pression et disposée en amont du dialyseur ou du filtre, et **en ce que** le signal de pression à retourner à la pompe à substitut est obtenu à partir du signal de pression oscillant et se propageant dans le circuit sanguin, mesuré en aval du dialyseur ou du filtre, un apport en liquide de substitution en amont ou en aval du dialyseur ou du filtre étant détecté sur la base de la comparaison des signaux de pression oscillants de la pompe à sang et de la pompe à substitut.

3. Procédé selon la revendication 2, **caractérisé en ce qu'**une analyse de Fourier du signal de pression mesuré en aval du dialyseur ou du filtre est effectuée pour obtenir les signaux de pression oscillants de la pompe à sang et de la pompe à substitut.

4. Procédé selon la revendication 3, **caractérisé en ce que** l'amplitude d'au moins deux composantes harmoniques de la pompe à substitut est déterminée à partir du signal de pression de la pompe à substitut, et l'amplitude d'au moins deux composantes harmoniques de la pompe à sang à partir du signal de pression de la pompe à sang, **en ce qu'**au moins un premier facteur d'évaluation est déterminé à partir d'au moins un quotient d'au moins une composante harmonique d'ordre supérieur et d'au moins une composante harmonique d'ordre inférieur de la pompe à substitut, et **en ce qu'**au moins un deuxième facteur d'évaluation est déterminé à partir d'au moins un quotient d'au moins une composante harmonique d'ordre supérieur et d'au moins une composante harmonique d'ordre inférieur de la pompe à sang, et **en ce que** le ou les premiers, et le ou les deuxièmes facteurs d'évaluation sont comparés entre eux.

5. Procédé selon la revendication 4, **caractérisé en ce qu'**il est conclu à un apport en liquide de substitution en aval du dialyseur ou du filtre si la différence entre le premier facteur d'évaluation et le deuxième facteur d'évaluation est supérieure à une valeur limite définie.

6. Procédé selon la revendication 4, **caractérisé en ce qu'**il est conclu à un apport en liquide de substitution en amont du dialyseur ou du filtre si la différence entre le premier facteur d'évaluation et le deuxième facteur d'évaluation est inférieure à une valeur limite définie.

7. Appareil de traitement extracorporel du sang, avec un circuit sanguin extracorporel (5A) qui comprend un premier compartiment (3) d'un dialyseur (1) ou d'un filtre subdivisé par une membrane (2) en ledit premier compartiment et en un deuxième compartiment (4), et un système de liquide (5B) qui comprend ledit deuxième compartiment du dialyseur ou du filtre, une conduite de liquide de substitution (17, 19, 20) où est disposée une pompe à substitut (22) générant des ondes de pression débouchant dans le circuit sanguin extracorporel (5A) en amont ou en aval du dialyseur (1) ou du filtre, et

   * un dispositif de détection de l'apport en liquide de substitution en amont ou en aval du dialyseur (1) ou du filtre,

   **caractérisé en ce que** le dispositif de détection de l'apport en liquide de substitution comprend :

■ une unité de mesure (28) pour mesurer la pression du circuit sanguin extracorporel (5A) en aval du dialyseur (1) ou du filtre, et
■ une unité d'analyse (50), comprenant :

■ des moyens (50a) pour l'obtention du signal de pression oscillant sur la pompe à substitut (22) à partir du signal de pression oscillant et se propageant dans le circuit sanguin, mesuré en aval du dialyseur (1) ou du filtre,
■ des moyens (50b) pour la comparaison du signal de pression oscillant de la pompe à substitut (22) avec un signal de référence caractéristique, et
■ des moyens (50c) pour la détection de l'apport en liquide de substitution en amont ou en aval du dialyseur (1) ou du filtre, sur la base de la comparaison du signal de pression oscillant de la pompe à substitut (22) avec le signal de référence caractéristique.

8.  Appareil selon la revendication 7, **caractérisé en ce qu'**une pompe à sang (10) générant des ondes de pression est disposée dans le circuit sanguin (5A) en amont du dialyseur (1) ou du filtre pour refouler le sang, et **en ce que** l'unité d'analyse (50) comprend en outre :

des moyens (50a) pour l'obtention du signal de pression oscillant sur la pompe à sang (10) à partir du signal de pression oscillant et se propageant dans le circuit sanguin, mesuré en aval du dialyseur (1) ou du filtre,
les moyens (50c) pour la détection de l'apport en liquide de substitution en amont ou en aval du dialyseur (1) ou du filtre étant réalisés de telle manière que l'apport du liquide de substitution est détecté en amont ou en aval du dialyseur ou du filtre sur la base de la comparaison du signal de pression oscillant de la pompe à substitut (22) avec le signal de pression de la pompe à sang (10).

9.  Appareil selon la revendication 8, **caractérisé en ce que** les moyens pour l'obtention des signaux de pression oscillants de la pompe à substitut (22) et de la pompe à sang (10) comprennent un dispositif d'analyse de Fourier (50a) du signal de pression mesuré en aval du dialyseur (1) ou du filtre.

10. Appareil selon la revendication 9, **caractérisé en ce que** le dispositif d'analyse de Fourier (50a) est réalisé
de telle manière que l'amplitude d'au moins deux composantes harmoniques de la pompe à substitut est déterminée à partir du signal de pression oscillant de la pompe à substitut (22), et l'amplitude d'au moins deux composantes harmoniques de la pompe à sang est déterminée à partir du signal de pression oscillant de la pompe à sang (10), au moins un premier facteur d'évaluation étant déterminé à partir d'au moins un quotient d'au moins une composante harmonique d'ordre supérieur et d'au moins une composante harmonique d'ordre inférieur de, la pompe à substitut (22), et au moins un deuxième facteur d'évaluation étant déterminé à partir d'au moins un quotient d'au moins une composante harmonique d'ordre supérieur et d'au moins une composante harmonique d'ordre inférieur de la pompe à sang (10), et
**en ce que** les moyens (50b) pour la comparaison du signal de pression oscillant sont réalisés de telle manière que le ou les premiers, et le ou les deuxièmes facteurs d'évaluation sont comparés entre eux.

11. Appareil selon la revendication 10, **caractérisé en ce que** les moyens (50c) pour la détection de l'apport en liquide de substitution sont réalisés de telle manière qu'il est conclu à un apport en liquide de substitution en aval du dialyseur (1) ou du filtre si la différence entre le premier facteur d'évaluation et le deuxième facteur d'évaluation est supérieure à une valeur limite définie.

12. Appareil selon la revendication 10, **caractérisé en ce que** les moyens (50c) pour la détection de l'apport en liquide de substitution sont réalisés de telle manière qu'il est conclu à un apport en liquide de substitution en amont du dialyseur (1) ou du filtre si la différence entre le premier facteur d'évaluation et le deuxième facteur d'évaluation est inférieure à une valeur limite définie.

EP 2 108 391 B1

**Fig. 1**

Fig. 2A

Fig. 2B

**Venenseitiger Druckverlauf während In Vitro HDF Dialysebehandlung**

Dialysegerät: 4008    Uf-Rate: 0 ml/h    Blutfluss: 250 ml/min    Pre/Post Sub-Rate: 70 ml/min

HDF Post-Dilution

HDF Pre-Dilution

mittlerer venöser Druck [mmHg]

Zeit [min]    **Fig. 3A**

Zeit [min]    **Fig. 3B**

EP 2 108 391 B1

**Fig. 4**

## Fouriertransformierte:

| Norm. Koeffizienten der Signale der arteriellen Blutpumpe | 0,276 |
|---|---|
| Norm. Koeffizienten der Signale der Substituatpumpe | 0,403 |
| Auswertung | 0,403>>0,276=>post |

**Fig. 5A**

## Fouriertransformierte:

| Norm. Koeffizienten der Signale der arteriellen Blutpumpe | 0,265 |
|---|---|
| Norm. Koeffizienten der Signale der Substituatpumpe | 0,177 |
| Auswertung | 0,265≥0,177=>pre |

**Fig. 5B**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0189561 A
- EP 1348458 A1
- DE 10115991 C1